(19) Europäisches Patentamt

European Patent Office

Office européen des brevets

(11) Numéro de publication: **0 582 147 A2**

(12) **DEMANDE DE BREVET EUROPEEN**

(21) Numéro de dépôt: **93111789.9**

(22) Date de dépôt: **23.07.93**

(51) Int. Cl.⁵: **A61K 7/48**, A61K 7/32, A61K 35/78

(30) Priorité: **03.08.92 EP 92113188**

(43) Date de publication de la demande:
**09.02.94 Bulletin 94/06**

(84) Etats contractants désignés:
**AT BE CH DE DK ES FR GB GR IE IT LI NL PT SE**

(71) Demandeur: **SOCIETE DES PRODUITS NESTLE S.A.**
**Case postale 353**
**CH-1800 Vevey(CH)**

(72) Inventeur: **Huynh-Ba, Tuong**
**5b, ch. de Leisis**
**CH-1009 Pully(CH)**
Inventeur: **Nguyen, Tuan**
**21, ch. du Verger**
**CH-1052 Le Mont-sur-Lausanne(CH)**
Inventeur: **Philippossian, Georges**
**18, Av. de Valmont**
**CH-1010 Lausanne(CH)**
Inventeur: **Vanstraceele, Anne**
**Parc du Manoir 129**
**F-60270 Gouvieux(FR)**

(74) Mandataire: **Archambault, Jean et al**
**55, avenue Nestlé**
**CH-1800 Vevey (CH)**

(54) **Composition cosmétique ou dermatologique anti-uréase.**

(57) Une composition cosmétique ou dermatologique anti-uréase, notament anti-transpirante et anti-érythème fessier contient un tanin condensé de caroube ou un dérivé d'acide chlorogénique en quantité efficace à titre d'agent anti-uréase en combinaison avec un support cosmétique approprié.

EP 0 582 147 A2

L'invention concerne une composition cosmétique ou dermatologique anti-uréase.

La formation d'ammoniac lors de la dégradation de l'urée par l'uréase joue un rôle important dans la transpiration et l'érythème fessier. Dans le cas particulier de l'érythème fessier, la formation d'ammoniac n'est pas la cause directe de celui-ci mais entraine une augmentation du pH favorisant l'augmentation de l'activité des lipases et protéases fécales, entrainant ainsi une irritation au niveau cutané.

De nombreuses voies ont été cherchées pour remédier au problème de l'érythème fessier.

On a introduit des couches jetables assurant une absorption maximale. Les produits pharmaceutiques ou cosmétiques disponibles sur le marché ont pour but d'apaiser la dermite fessière. Ces derniers contiennent des agents protecteurs (l'utilisation de certains de ces produits peut être dangereuse), des antiseptiques, des anti-inflammatoires (délivrés exclusivement sur ordonnance) ou des systèmes tampon (simple neutralisation de l'ammoniaque et des acides issus des dégradations bactétiennes).

Cependant, les moyens connus mentionnés ci-dessus se bornent à atténuer les effets de l'activité des enzymes sans s'attaquer à la cause de cette activité.

Par ailleurs, on connait certains agents anti-uréase contre l'érythème fessier et comme déodorant, par exemple les acides hydroxamique (par exemple dans JP-A-57104276) et hydroximique (par exemple dans JP-A-57119997). D'une manière plus générale, on connait l'activité anti-uréase de polyhydroxyphénols et de quinones (par exemple dans "H.J.Michel, die Pharmazie, Heft 2, Februar 1980, S. 66, Abs. 6"), sans qu'il n'y soit cependant fait mention d'une application dans le traitement de l'érythème fessier ou comme anti-transpirant.

Le but de l'invention est l'élaboration d'une composition anti-transpirante ou anti-érythème fessier dont le principe actif est d'origine naturelle ou synthétique et dans ce dernier cas est un composé trouvé dans la nature et resynthétisé. Dans le cas d'une composition anti-érythème fessier, le but est également de lui conférer un aspect et un toucher plus agréables que ceux des compositions anti-érythémateuses connues du type des pommades pharmaceutiques.

L'invention concerne donc une composition cosmétique ou dermatologique anti-uréase contenant des tannins, caractérisée par le fait qu'elle contient une quantité efficace d'un agent anti-uréase choisi parmi les tanins condensés de caroube et les dérivés de l'acide chlorogénique.

Dans le contexte de l'invention, les tanins condensés de caroube sont constitués d'esters d'acide gallique ou de formes plus complexes de l'acide gallique faisant intervenir des liaisons depsidiques (liaison entre 2 galloyles) et de glucose. Les tanins condensés d'Hamamélis sont constitués d'esters d'acide gallique ou de formes plus complexes de l'acide gallique précédentes et d'hamamélose. Ces tanins peuvent être sous forme d'extraits végétaux plus ou moins purifiés.

Par "dérivés de l'acide chlorogénique" on entend les acides caféoyl-, dicaféoyl-, féruoyl-, coumaroyl- et caféoylféruoyl-quiniques ainsi que les quinides (lactones d'acide quinique) correspondantes. Ces dérivés peuvent être sous forme d'extraits naturels de plantes, par exemple de café ou d'artichaut ou sous forme synthétique. Une source de choix de ces acides chlorogéniques est constituée par un résidu de la décaféination d'un extrait aqueux de café vert par adsorption, par exemple sur charbon actif. Ce résidu est obtenu par désorption de l'adsorbat par élution à partir de l'adsorbant, puis élimination de la caféine de l'éluat par extraction liquide/liquide.

Selon l'invention, on entend par quantité efficace de l'agent anti-uréase celle qui procure une inhibition de 50% de l'activité de l'uréase mesurée par la méthode indiquée ci-après dans l'exemple 1. Cette inhibition est obtenue avec une quantité de 200 à 2000 microg d'agent anti-uréase.

Une composition cosmétique selon l'invention peut se présenter sous une forme de solution ou de dispersion aqueuse ou non, ou de préférence d'émulsion eau-dans-l'huile ou huile-dans-l'eau. Elle comprend de préférence 0,1 à 10% en poids d'agent anti-uréase en combinaison avec un support cosmétique tel que par exemple une lotion, une crème ou un lait.

Dans une émulsion, la phase grasse peut contenir des huiles animales, végétales, minérales ou synthétiques. elle peut également contenir des cires, des alcools à longue chaîne, des agents épaississants ou gélifiants. Une composition sous forme d'émulsion contient de préférence 1 à 20% en poids d'un agent émulsifiant.

Une composition selon l'invention peut contenir en outre divers additifs, notamment des agents colorants, des parfums, des agents conservateurs, des agents humectants, des agents tampons, des agents nacrants et des charges minérales ou organiques.

Elle contient avantageusement des antioxydants, de préférence à raison de 0,02 à 0,2% en poids.

L'invention concerne également l'utilisation d'un extrait d'Hamamélis à titre d'agent anti-uréase.

Dans une telle utilisation, l'extrait d'Hamamélis peut être incorporé dans une composition cosmétique ou dermatologique telle que décrite précédemment.

Les exemples ci-après illustrent l'invention. Dans ceux-ci, les parties et pourcentages sont pondéraux sauf indication contraire.

Exemple 1

On détermine l'activité anti-uréase des composés inhibiteurs utilisables selon l'invention à différentes concentrations.

Méthode

La méthode d'analyse de l'activité anti-uréase d'un inhibiteur est basée sur le principe du dosage de l'ammoniac.

L'urée contenue dans un échantillon de substrat contenant un mélange d'inhibiteur, d'urée et d'uréase, est dégradée par l'uréase à 37°C pendant une durée d'incubation imposée de 15 min. Ces deux derniers réactifs sont dans les proportions suivantes: 7 microg d'urée pour 25 microg d'uréase (d'origine végétale, de jack bean), soit 0,25 Unité, c'est à dire 0,25 ml d'une solution de 25 mg d'uréase dissous dans 25 ml de solution tampon, elle-même préparée par dissolution de 6 g d'acide 4(2-hydroxyéthyl)-pipérazine 1 éthanesulfonique et de 0,2 g d'éthylènediaminetétraacétate dans 1 l d'eau et ajustement du pH à 7,5 par addition d'une solution diluée d'hydroxyde de sodium). L'ammoniac issu de la réaction enzymatique forme quantitativement un dérivé d'indophénol avec les réactifs hypochlorite de sodium, phénol et nitroprussiate de sodium.

On ajoute ainsi à l'échantillon 4 ml de solution préparée par dissolution de 10 g de phénol et 50 mg de nitroprussiate de sodium dans 1 l d'eau, puis on complète à 10 ml par addition d'une solution d'hypochlorite de sodium (préparée par dissolution de 5 g d'hydroxyde de sodium et 10 ml d'hypochlorite de sodium dans 1 l d'eau).

Après une nouvelle incubation à 37°C pendant 20 min, on mesure l'adsorbance par spectrophotométrie par comparaison avec le même mélange de réactifs sans échantillon, le dérivé d'indophénol obtenu étant doté d'une coloration dont l'intensité est proportionnelle à la quantité d'ammoniac libérée et mesurable à 634 nm dans des cellules de 10 mm. Le % d'ammoniac libéré est exprimé par le rapport: densité optique de l'échantillon avec inhibiteur / densité optique de l'échantillon sans inhibiteur x 100.

Résultats

On exprime l'activité anti-uréase comme la quantité d'inhibiteur (en microg) nécessaire pour obtenir une inhibition de 50%, représentée par la valeur moyenne de 4-5 déterminations pour chaque inhibiteur testé.

Les résultats sont consignés dans le tableau 1 ci-après.

**Tableau 1**

| Inhibiteur | Activité (microg) |
|---|---|
| Acide usnique (extrait glycolique de Usnea barbata à 7,5% d'usnate) | 250 |
| Acide chlorogénique (extrait d'artichaut à 46,7% d'acide chlo-rogénique) | 200 |
| Hamamélis (à 15% de tanins) | 200 |
| Résidu de la décaféination du café vert élué à partir de la fraction retenue sur charbon actif | 500 |
| Extrait de caroube verte (désucré à l'eau chaude) | 500 |
| Extrait de thé vert | 1000 |
| 5-CQA | 250 |
| 1-CQA | 250 |
| 3,4-di-CQA | 250 |
| 3-CoQA | 350 |
| 3-FQA | 1000 |
| 3,4-di-CoQA | 1000 |

**Tableau 1 (suite)**

| Inhibiteur | Activité (microg) |
|---|---|
| 3,4-di-FQA | 1000 |
| 3,4-di-CFQA | 1500 |

**Légende**

CQA = acide caféoyl quinique

FQA = acide féruloyl quinique

CoQA = acide coumaroyl quinique

CFQA = acide caféoylféruloyl quinique

Les dérivés de l'acide quinique précédents ont été préparé selon les demandes de brevet de la titulaire déposées le même jour que la présente demande sous les titres "Dérivés de l'acide quinique et procédé de préparation de dérivés de l'acide quinique" et "Dérivés de l'acide quinique 3- et/ou 4-substitués et procédé de préparation de dérivés de l'acide quinique 3- et/ou 4-substitués"

Exemple 2

On réalise un test in vitro d'une crème cosmétique protectrice dont on évalue l'activité anti-uréase comme indiqué à l'exemple 1 par détermination colorimétrique de l'ammoniac libéré et dont on mesure le pH directement dans les mélanges réactionnels au moyen d'une électrode de verre. L'hypothèse de départ est qu'un bébé de poids moyen 6 kg est langé toutes les 4 h. Pendant ces 4 h il produit en moyenne 40 ml d'urine correspondant à 300 mg d'urée et 30 g de fèces contenant une quantité d'uréase variant entre 3 et 30 U. Ainsi la quantité de crème à appliquer sur les parties sensibles est évaluée à 10 g contenant 400 mg d'inhibiteur extrait d'Amamelis.

La crème est une émulsion huile-dans-l'eau dont la composition est indiquée dans le tableau 2 ci-après (la nomenclature utilisée pour les ingrédients est celle de la "Cosmetic, Toiletery and Flagrance Association, Inc. Washington DC", mise à part la traduction en français).

## Tableau 2

| Ingrédient | % |
|---|---|

**Phase A**

| | |
|---|---|
| Stéarate de glycérol | 10 |
| Octanoate de cétéaryl | 3 |
| Oléine de palme (contenant des acides gras en C12-C14) | 3 |
| Huile de tournesol | 4 |
| Huile de germe de blé | 0,3 |

**Phase B**

| | |
|---|---|
| Glycérine | 5 |
| Eau | qsp 100 |

**Phase C**

| | |
|---|---|
| Cyclométhicone | 3 |
| Acétate de DL-alpha-tocophérol | 0,2 |
| Conservateur | 0,2 |
| Extrait d'Hamamélis | 4 |

Pour fabriquer la crème, on chauffe séparément les phases A et B à 80°C, puis on verse la phase B dans la phase A sous forte agitation. On laisse refroidir ensuite le mélange jusqu'à 40°C sous agitation constante, on y incorpore la phase C sous vive agitation, puis on laisse refroidir jusqu'à la température ambiante sous agitation constante.

On détermine l'activité de la crème avec l'inhibiteur par comparaison avec la crème dépourvue d'inhibiteur aux concentrations 0,75 U et à 7,5 U d'enzyme. Les résultats relatifs à la quantité d'ammoniac libérée sont indiqués dans le tableau 3 et au pH dans le tableau 4 ci-après.

Tableau 3

| Composition | Quantité d'enzyme (U) | Ammoniac libéré (mg) après une incubation d'une durée X (h) | | | | |
|---|---|---|---|---|---|---|
| | | 1/4 | 3 | 7 | 20 | 24 |
| Crème avec 4% d'extrait d'Hamamélis | 0,75 | 0,47 | 0,89 | 0,97 | 0,97 | 0,92 |
| | 7,5 | 1,13 | 1,04 | 1,27 | -- | 0,87 |
| Crème sans inhibiteur | 0,75 | 1,38 | 2,18 | 1,74 | 2,26 | 2,61 |
| | 7,5 | 5,92 | 9,66 | 9,66 | -- | 9,38 |

6

Tableau 4

| Composition | Quantité d'enzyme (U) | pH après une durée X (h) | |
|---|---|---|---|
| | | 0 | 24 |
| Crème avec | 0,75 | 5,79 | 5,84 |
| 4% d'extrait d'Hamamélis | 7,5 | 5,78 | 5,83 |
| Crème sans | 0,75 | 5,94 | 6,53 |
| inhibiteur | 7,5 | 5,94 | 7,73 |

Les résultats obtenus montrent que la crème contenant l'extrait d'Hamamélis est capable d'inhiber totalement l'uréase aux deux concentrations de 3 et 30 U.

Exemple 3

On prépare une crème protectrice sous forme d'une émulsion eau-dans-l'huile dont la composition est indiquée dans le tableau 5 ci-après.

## Tableau 5

| Ingrédient | % |
|---|---|
| **Phase A** | |
| Cire microcristalline, | 9 |
| cocoate de pentaerythrol, | |
| citrate de stéaryl, | |
| oléate de glycérol, | |
| stéarate d'aluminium et | |
| propylène glycol | |
| Oléate de décyl | 3 |
| Oléine de palme (contenant | 10 |
| des acides gras en C12-C14) | |
| Huile de tournesol | 12 |
| Ozokérite | 0,5 |
| Huile de germe de blé | 0,3 |
| | |
| **Phase B** | |
| Glycérine | 5 |
| Eau | qsp 100 |
| | |
| **Phase C** | |
| Acétate de DL-alpha-tocophérol | 0,2 |
| Cyclométhicone | 3 |
| Conservateur | 0,2 |
| Extrait d'Hamamélis | 4 |

On prépare la crème huile dans l'eau comme indiqué précédemment pour la crème de l'exemple 2.

## Revendications

1. Composition cosmétique ou dermatologique anti-uréase contenant des tanins, caractérisée par le fait qu'elle contient une quantité efficace d'un agent anti-uréase choisi parmi les tanins condensés de caroube et les dérivés de l'acide chlorogénique.

2. Composition anti-transpirante et anti-érythème fessier selon la revendication 1.

3. Composition selon la revendication 1, caractérisée par le fait qu'elle contient un résidu de la décaféination d'un extrait aqueux de café vert riche en acide chlorogénique à titre d'agent anti-uréase.

4. Composition selon l'une des revendications 1 à 3, caractérisé par le fait qu'elle contient 0,1 à 10% en poids d'agent anti-uréase.

5. Composition selon la revendication 4, caractérisée par le fait qu'elle se présente sous forme d'une crème constituée d'une émulsion eau dans l'huile ou huile dans l'eau.

6. Procédé de préparation d'une composition selon la revendication 5, caractérisé par le fait que l'on chauffe séparément une phase grasse contenant un émulsifiant et une phase aqueuse, que l'on mélange les deux phases à chaud sous forte agitation, qu'on laisse refroidir le mélange sous agitation jusqu'à une température intermédiaire, que l'on y incorpore des additifs et l'agent anti-uréase, puis que l'on refroidit la mélange jusqu'à la température ambiante sous agitation constante.

7. Utilisation d'un extrait d'Hamamélis à titre d'agent anti-uréase.

8. Utilisation selon la revendication 7, à titre d'agent anti-érythème fessier.